(19) Europäisches Patentamt
European Patent Office
Office européen des brevets

(11) **EP 4 255 309 B1**

(12) **EUROPEAN PATENT SPECIFICATION**

(45) Date of publication and mention
of the grant of the patent:
**11.09.2024  Bulletin 2024/37**

(21) Application number: **21830331.1**

(22) Date of filing: **24.11.2021**

(51) International Patent Classification (IPC):
*A61B 8/08* *(2006.01)*      *A61B 8/00* *(2006.01)*

(52) Cooperative Patent Classification (CPC):
**A61B 8/4477; A61B 8/0825; A61B 8/406;**
**A61B 8/4209; A61B 8/4281; A61B 8/4461;**
**A61B 8/4494; A61B 8/483**

(86) International application number:
**PCT/IB2021/060914**

(87) International publication number:
**WO 2022/118145 (09.06.2022 Gazette 2022/23)**

(54) **DEVICE AND METHOD FOR BREAST CANCER DIAGNOSIS**

VORRICHTUNG UND VERFAHREN ZUR BRUSTKREBSDIAGNOSE

DISPOSITIF ET PROCÉDÉ DE DIAGNOSTIC DE CANCER DU SEIN

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **01.12.2020   IT 202000029309**

(43) Date of publication of application:
**11.10.2023  Bulletin 2023/41**

(73) Proprietor: **Imedicals Srl**
**73100 Lecce (IT)**

(72) Inventor: **CASCIARO, Sergio**
**73100 Lecce (IT)**

(74) Representative: **Conversano, Gabriele**
**Laforgia, Bruni & Partners srl**
**Via Michele Garruba, 3**
**70122 Bari (IT)**

(56) References cited:
**EP-A1- 2 868 279        US-A1- 2013 190 595**
**US-A1- 2018 256 130**

**Description**

Technical field

The present invention relates to a device for carrying out an examination for breast cancer diagnosis.

More in particular, the present invention relates to a device allowing to reconstruct both a 3D ultrasound model of the breast volume and information about the frequency spectra and the acoustic attenuation coefficient in a perfectly overlapping way, and to calculate a diagnostic parameter indicating the presence of a breast carcinoma according to these data.

State of the art

Many problems are linked to the breast ultrasound examination.

*In primes*, it is desirable to guarantee the repeatability of the examination, and in particular of the ultrasound probe (or probes) positioning with respect to the breast, regardless of the operator manual skills and expertise; *in secundis*, if the breast is to be 3D reconstrued it is needed to guarantee that the breast is in a well determined position with respect to the ultrasound probe (or ultrasound probes) while this one is moved to scan a plurality of positions, obviously guaranteeing the presence of a suitable acoustic coupling means (water, gel or other suitable material) between the probe and the skin.

Finally, once the above cited technical problems are solved, it is needed to analyze the ultrasound data collected by means of a method allowing high accuracy in breast cancer diagnosis.

Many devices and methods are known for ultrasonic and/or ultrasound data analysis aiming at diagnosing breast cancer, that try to solve said problems.

CN110680380 describes a device using a ring with a plurality of ultrasound transducers arranged thereon, which is positioned outside a cylindrical container inside which the breast of a patient, laying prone, is immersed in water. The device is used to produce ultrasound images according to which the doctor can make a diagnosis of breast cancer.

WO02089672 described an apparatus in which a linear array of piezoelectric transducers is arranged parallel to the axis of symmetry of the breast, and is configured to rotate around the same, inside a container in which a coupling liquid is provided.

EP2868279 describes a device for breast ultrasound using a plurality of transducers arranged on a supporting element.

US2013/0041261 describes a device using a ringshaped transducer, which comprise a plurality of 2 MHz frequency ultrasound senders and receivers, configured to determine a spatial distribution of breast acoustic and mechanical parameters.

US2012029358 shows a device using a plurality of ultrasound probes, arranged symmetrically around the axis of symmetry of the breast, configured for imaging, at frequencies between 7.5 and 10 MHz, in a plurality of planes going through the axis of symmetry of the breast.

US2004064046 described a device for breast ultrasound tomography comprising a stationary chamber for containing a fluid, inside which a mobile chamber is provided, which is integral to an array of ultrasound transducers and receivers.

Other examples are shown in CN111213065, US2013041260, US4222274, US10285667.

Technical problem

At the best of the present inventors' knowledge, all the just described devices, and the other ones known at the state of the art, have limits, since they do not allow to realize, during the same examination, a perfectly overlapped volumetric reconstruction both of the breast image and of the distribution of other characteristic parameters of the analyzed tissues, such for example the acoustic attenuation coefficient.

Moreover, no one of the known devices allows to calculate a diagnostic parameter that considers explicitly both piece of information (information obtainable from the ultrasound imaging and information obtainable from the analysis of acoustic attenuation coefficients).

Yet, no one of the devices known at the state of the art allows to calculate a diagnostic parameter that considers explicitly also the frequency analysis of the associated raw ultrasound signal. It is to be specified that, also in the following, for raw ultrasonic signal it is intended a radiofrequency ultrasonic signal, as it is received by the probe, before the various filtrations and processing necessary for obtaining the ultrasound image.

Aim of the invention

Aim of the present invention is to provide a device for carrying out an examination for breast cancer diagnosis, which overcomes the limits linked to the embodiments known at the state of the art, and in particular which allows to realize, during the same examination, a perfectly overlapping volumetric reconstruction both of the breast image and of the distribution of characteristic parameters of the analyzed tissues, such for example the acoustic attenuation coefficient.

According to another aim, the device object of the present invention comprises computing means on which computer programs are loaded to calculate a diagnostic parameter that considers explicitly both piece of information (information obtainable from the ultrasound imaging and information obtainable from the analysis of acoustic attenuation coefficients).

Yet, another aim of the present invention is to calculate a diagnostic parameter that considers explicitly also the parameters deriving from the frequency analysis of the ultrasound signals associated to specific portions of breast tissue.

Description of the figures

Figures 1, 2 and 3 show three views of a preferred and not limiting embodiment of the device;
Figure 4 shows a schematic view of a breast section along a generic plane of acquisition passing through the axis of symmetry of the device;
Figure 5 shows a section schematic view of a portion of tissue along a plane orthogonal to the axis of symmetry of the device;
Figure 6 shows a schematic view of a breast half section along a generic plane of acquisition passing through the axis of symmetry of the device, and figure 7 shows schematically: the shape of the signal received in transmission, in the time domain (a) and in the frequency domain (b), and the shape of the signal received in reflection, in the time domain (c) and in the frequency domain (d).

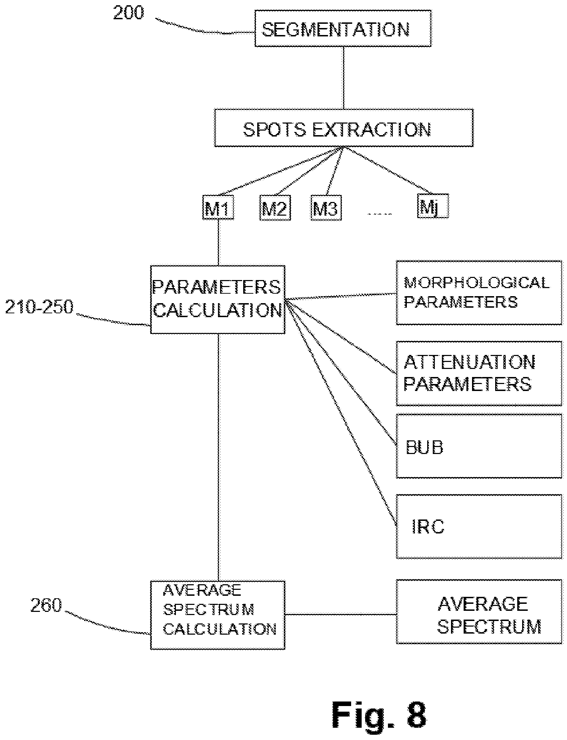

Figures 8 and 9 show examples of flowcharts of a preferred embodiment of the method which can be carried out by means of the device according to the invention;
Figure 10 shows a schematic section view of a probe, highlighting the mechanic coupling means;

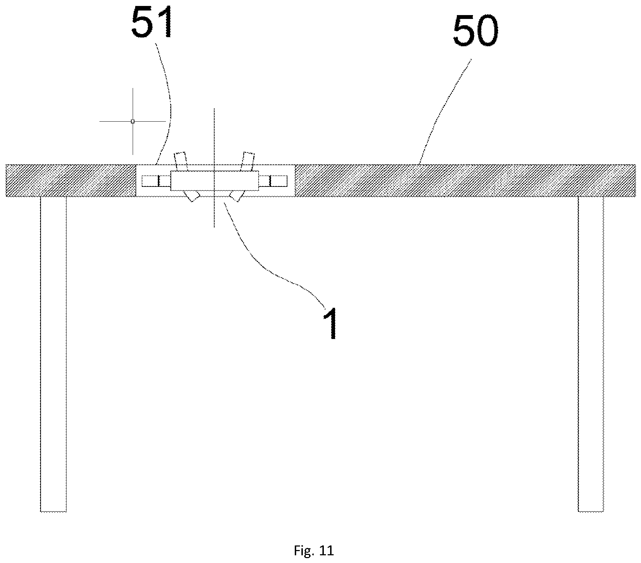

Figure 11 shows the device installed under an examination table;

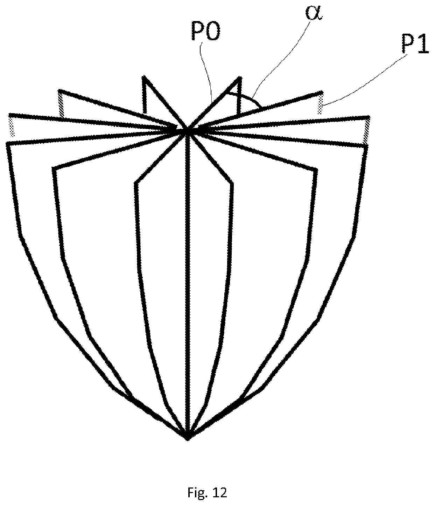

Figure 12 shows a tridimensional schematization of the arrangement of various planes of acquisition.

Detailed description

Before the following description, it is to be specified that:

- for tumor tissue it is intended the tissue relating to a suspect area for which the presence of a tumor has been verified by means of histologic examination or by means of the method according to the present invention;
- for not tumoral tissue it is intended the tissue relating to a suspect area for which the presence of a tumor has been excluded by means of histologic examination or by means of the method according to the invention. In other words, the not tumoral tissue relates to an inhomogeneity detectable by ultrasound or by means of ultrasound analysis, resulted benign thereupon;
- for healthy tissue it is intended the tissue with no inhomogeneity characteristics detectable by ultrasound or by means of ultrasound analysis, and not classified as suspect area.

It is also to be specified at first that the realization of the device is described with reference to the use of piezoelectric transducers, but other types of transducers can be also used, as for example CMUT capacitive transducers, without departing from the aims of the invention.

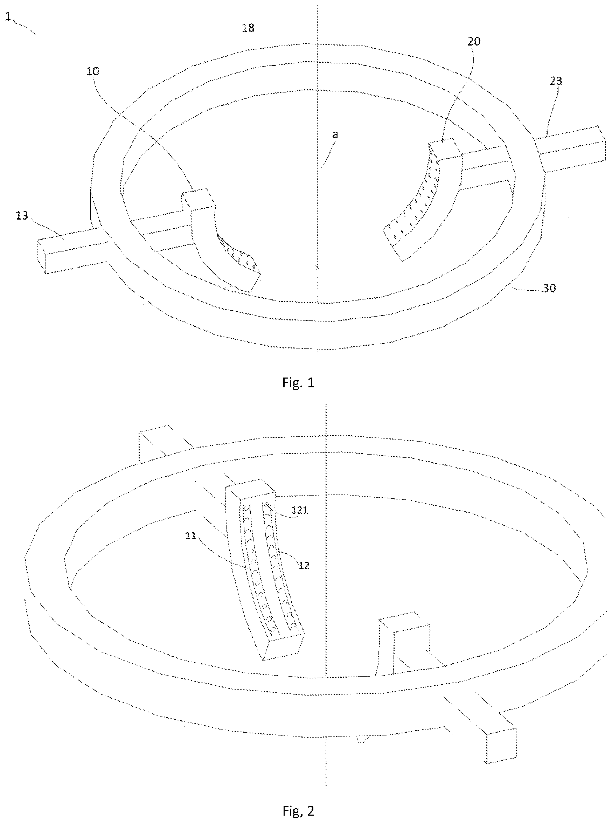

With reference to figure 1, the device (1) according to the invention comprises two ultrasound probes (10, 20), each one being associated to two arrays of piezoelectric transducers (11, 12, 21, 22).

In a first embodiment, said arrays are linear arrays. In a second embodiment, said arrays are concave, so that the concavity allows a better breast shape adherence. It is to be specified that the concavity is associated to the outer shape of the probe head.

Each of the two ultrasound probes (10, 20) comprises a first array of piezoelectric transducers (11, 21) with a first nominal frequency or band center frequency (f1), and a second array of piezoelectric transducers (12, 22) with a second nominal frequency or band center frequency (f2).

Said first nominal frequency (f1) is chosen to carry out a B-Mode ultrasound imaging starting from the signals reflected by breast tissues and is preferably but not limitingly between 7 and 10 MHz. From the following description, it will be clear that each probe is used for high resolution ultrasound imaging acquisition of the breast portion comprised between the skin and the axis of symmetry thereof. For this reason, considering that the depth of the analyzed tissue is limited, high frequencies can be used without the attenuation associated thereto becomes a problem.

Said second nominal frequency (f2) is chosen to penetrate better the tissue, and so to allow acoustic attenuation measures working in transmission mode with the array of the other probe with the same nominal frequency. Said second nominal frequency (f2) is preferably between 1 and 3 MHz.

The two arrays (12, 22) with the lowest nominal frequency (f2) are provided with the same number of piezoelectric transducers, so that a respective transducer (221) of the array (22) of the second probe (20) corresponds to each transducer (121) of the array (12) of the first probe (10).

The realization of each of the two probes is such that all the piezoelectric transducers associated thereto are rigidly fastened to each other. In other words, the relative position of the two arrays of piezoelectric transducers is fixed. In particular, each probe comprises two arrays, linear or concave next to each other, of piezoelectric transducers.

Each probe comprises also coupling means (16, 26) to a control device (not shown in figure) configured to guide said probes and to detect and analyze the signal acquired by the same, according to what described in detail in the following.

In a first preferred embodiment, each probe (10, 20) comprises also a fixing rod (13, 23). The two fixing rods (13, 23) are associated to a circular support (30) in diametrically opposed positions. In other words, the two probes (10, 20) are positioned along the same direction, opposite to each other, with the arrays (11, 12, 21, 22) positioned in the same plane of the axis of symmetry (a) of the circular guide (30), and symmetrical to the same.

Each of the two probes (10, 20) is positioned along a radial direction of said circular guide (30), and it is associated in the same way so that the fixing rod (13, 23) can slide, thus allowing the probe (10, 20) to be positioned at different distances from the axis of symmetry (a) of the circular guide (30) .

Preferably, the device comprises also thrusting means (not shown in figure 1) configured to thrust the two probes (10, 20) radially towards the center. Preferably, said thrusting means comprise at least a spring element, configured to thrust the respective probe (10, 20) towards the center.

The device comprises also movement means (40) configured to rotate said circular guide (30) around its own axis of symmetry (a).

The movement means (40) comprise one or more electric motors and respective control means. These are movement means known per se at the state of the art.

Regardless of the embodiment, the just described one being a preferred and not limiting variant thereof, the device (1) according to the invention comprises two ultrasound probes (10, 20), each comprising two arrays (11, 12, 21, 22) of piezoelectric transducers with two different nominal frequencies (f1, f2), said probes being arranged opposite to each other on the same straight line, and being configured so that they can rotate along a circular trajectory having for center the midpoint of the segment connecting them and so that they can slide to each other along the straight line connecting them.

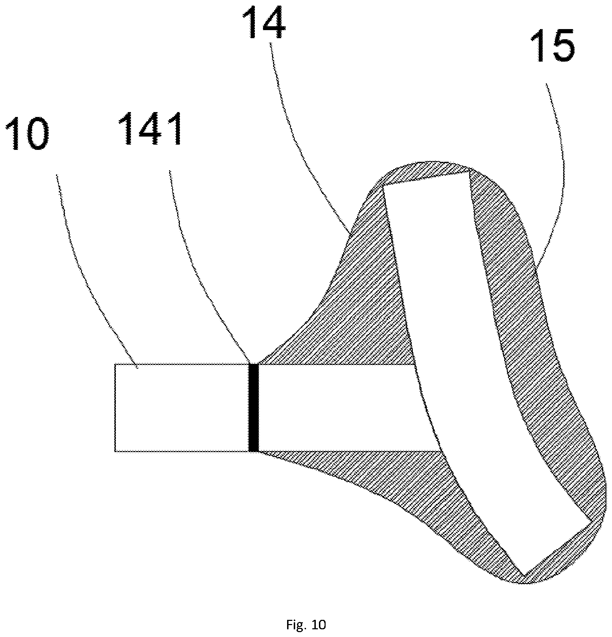

As it is shown in figure 10, moreover preferably each probe comprises also acoustic coupling means (14, 15) to the breast skin. In a first preferred embodiment, said acoustic coupling means comprise a flexible membrane (14), filled with a gel (15). The assembly of membrane and gel is configured to be pressed when the probe is thrusted towards the breast, thus adapting to the breast shape and thus guaranteeing the mechanic coupling with this latter for the ultrasound transmission.

Preferably, to such aim the breast is covered with gel, liquid or any other material suitable for the ultrasound transmission.

In a second embodiment, the device is immersed in a vessel containing water or any other liquid suitable for functioning as acoustic coupling means, and open in the upper portion so that the breast can be introduced from above.

The device (1) is configured to be positioned under an examination table (50), at an opening (51) in which the breast of the woman, laying prone on the same table, can be introduced.

After describing the device, it is now possible to describe its functioning.

Once the patient is positioned prone on the table (51), the device (1) is positioned with its own axis of symmetry (a) at the axis of symmetry of the breast, and the two probes (10, 20) are thrusted towards the axis of symmetry, until they come in contact with the breast of the patient from diametrically opposed portions in a first angular position (P0) .

According to another embodiment, the device can be fastened integrally to the lower portion of a table (51), at a hole obtained on the surface of the same in suitable position.

Now, a first scanning can be carried out, according to what described in detail in the following, and so the circular guide (30) is rotated of an angle ($\alpha$), up to bring the two ultrasound probes in position (P1), and the sequence of scanning and rotations is repeated up to complete a 180° rotation of the circular guide (30) and to come back with the two probes aligned in the first angular position (PO), but in inverted positions.

In this way, the whole breast volume is scanned.

It is to be specified that the control device of the probes and acquisition of the ultrasound signals detected by them is configured:

- to guide individually each piezoelectric transducer of each array of each probe;
- to detect the signals acquired by each piezoelectric transducer of each probe;
- to store the "raw" (also called "radiofrequency") ultrasound signals acquired by each transducer, thus making them available for next processing;
- to process the radiofrequency signals for obtaining B-mode ultrasound images;
- to associate the relative position of acquisition to each signal detected and to each image.

Clearly, for the just described procedure as well as for all the other ones described in the document, the device according to the invention comprises electronic computing means on which computer programs are loaded configured to carry out the relative movement procedures of the device, the data acquisition, data storing, data processing and diagnostic parameter calculation.

In each position (PC, P1,...) the acquisition procedure is the following:

100) acquisition of a first B-mode ultrasound image relating to the portion of tissues comprised between the first probe (10) and the axis of symmetry, by means of the first array (11) of the first probe (10), by sending a plurality of ultrasound signals and detection of relative ultrasound signals reflected by the breast tissues;

110) acquisition of a second B-mode ultrasound image relating to the portion of tissues comprised between the second probe (10) and the axis of symmetry, by means of the first array (21) of the second probe (20), by sending a plurality of ultrasound signals and detection of the relative ultrasound signals reflected by the breast tissues;

120) storage of the "raw" (or "radiofrequency") ultrasound signals, as detected by each probe (10, 20) and before any following processing, in particular before the processing for the relative B-mode image creation;

130) emission of an ultrasound pulse by means of the first piezoelectric transducer (121) of the second array (12) of the first probe (10), and detection of the relative ultrasound signal transmitted by means of the first piezoelectric transducer (221) of the second array (22) of the second probe (20) and the relative ultrasound signal reflected by means of the first piezoelectric transducer (121) of the second array (12) of the first probe (10);

140) repetition of step 130) for each couple of piezoelectric transducers of the second arrays (12, 22) of the first and second probe.

At the end of the acquisition procedure, for each angular position (P0, P1,...), for the following processing are then available:

- two ultrasound images, acquired in the same plane, and relating to the portions of tissue (P0_1, P0_2) comprised between the axis of symmetry and each probe, obtained from the analysis of the reflected signals acquired by each of the two arrays having the first nominal frequency;
- a plurality of couples of signals, made up of an emitted signal (which is known on the basis of the structural characteristics of the probe used and relative guide system, commonly known also as "beamformer") and of a signal received in transmission mode, each couple of signals relative to a couple of transducers of the arrays of the first and second probe having the second nominal frequency;

- a plurality of ultrasound signals reflected in the time domain acquired by the arrays of each probe having the second nominal frequency;
- a plurality of ultrasound signals in the time domain (110S), reflected by the breast tissues upon the pulses sent by the arrays having the first nominal frequency and associated to respective propagation segments (110).

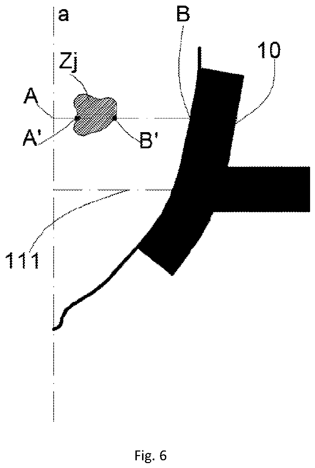

In figure 6, it is shown the path (110) of the ultrasound reflected signal detected by the first transducer of the first array (11) of the first ultrasound probe (10). For schematic purpose, figure 6 shows only the portion of tissue comprised between the axis of symmetry and the probe (10).

As it is always clear from the analysis of figure 6, which shows also the ultrasound reflected signal, to point A, positioned at greater distance from the probe, a signal corresponds that has an arrival time (tA) greater than the arrival time (tB) of the signal reflected from point B substantially arranged at the skin, in contact with the probe.

On the basis of this consideration, known per se, it is possible to associate the value of the corresponding reflected, raw (i.e. not processed) ultrasound signal to each point of the segment AB.

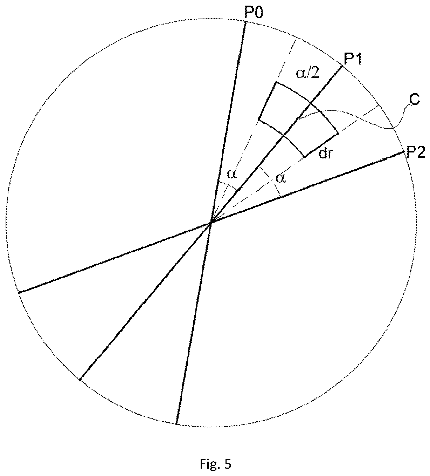

The assembly of the acquisitions carried out defines a volumetric grid of acquisition volumes, schematically shown in figure 5, where it is shown the plan projection of the $i^{th}$ (Vi) acquisition volume, associated to a generic point C.

So, the $i^{th}$ volume will have an angular amplitude (alfa) equal to the angle between two following positions of acquisition, a radial amplitude (dr) equal to the spatial resolution of the ultrasound imagining system in radial direction, i.e. in the sense of depth with respect to the ultrasound probe and a height (not shown in figure 5) corresponding to the "side spatial resolution" of the ultrasound image.

As it is known, the resolution of the ultrasound imagining system in the sense of depth corresponds theoretically to the half wave-length of the incident ultrasound pulse, which is inversely proportional to the frequency.

It is also to be specified that the angular amplitude of the volume acquired depends on the focusing of the used ultrasound beam. Conveniently, the angle ($\alpha$) between two following positions of acquisition (P0, P1) is chosen so that the whole volume is under acquisition and no areas are left uncovered.

So, to each $i^{th}$ volume, downwards of the acquisition

- a value ($S_i$) relating to the intensity of the reflected ultrasound image,
- a value ($B_i$) of the grayscale, associated to the B-mode ultrasound image

will be associated.

Moreover,

- the values of the acoustic attenuation coefficient calculated for each propagation line of the ultrasound signals emitted by said second array (12) of the first probe (10) and received by said second array (22) of the second probe (20);
- the acquisitions of the radiofrequency raw ultrasound signals, relating to all the propagation lines of the ultrasound signal of each array, for all the positions of acquisition

are available.

With the previously enlisted data, the method of analysis of the same to calculate a diagnostic parameter representing the presence of a breast cancer or not comprises the steps of:

200) segmentation of the breast volume 3d model to individuate the presence of one or more "suspect areas", characterized by clearer colour (hyperechoic) or darker colour (hypoechoic) with respect to the surrounding tissue, or anyway characterized by any other "inhomogeneity" form detectable by means of the grayscale analysis, which makes these areas "encirclable".

In case of individuation of one or more suspect areas ($Z_j$), calculation of a diagnostic parameter ($D_j$) referred to each area, indicating the probability that such area can be classified as breast cancer, said diagnostic parameter ($D_j$) depending on one or more of the following factors:

(i) morphological information deriving from the B-mode imaging of the points of the 3D model inside the $i^{th}$ area ($Z_j$);
(ii) information relating to the average acoustic attenuation coefficient relative to at least a portion of propagation line of the ultrasound signal contained inside the $j^{th}$ area ($Z_j$);
(iii) information relating to the frequency spectrum of the signal associated to the $j^{th}$ area ($Z_j$) or anyway derived by the analysis thereof, possibly carried out by means of quantitative comparisons with "model spectra" acquired on patients with known diagnoses.

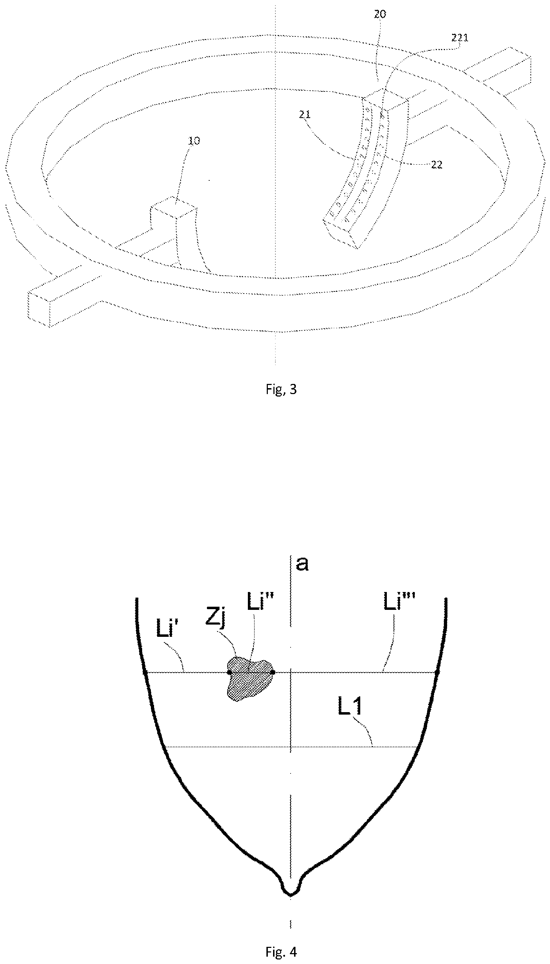

It is to be specified that, with reference to figure 4 (in which, for simplicity the propagation lines of the ultrasound signal are shown, but not the respective probes), the average acoustic attenuation coefficient relative to at least a portion of propagation line of the ultrasound signal contained in the $j^{th}$ area ($Z_j$) can be calculated:

- by calculating the acoustic attenuation coefficient of the healthy tissue (Ats), i.e. relative to a propagation line of the ultrasound signal (of L1 length) outside any suspect area individuated in the ultrasound image. Preferably, the acoustic attenuation coefficient of the healthy tissue (Ats) is calculated as the average of the acoustic attenuation coefficient calculated for a plurality of propagation lines of the ultrasound signal outside any suspect area detected during the ultrasound examination.
- So, for each propagation line (with whole length Li) passing through the $j^{th}$ area ($Z_j$), it is possible to calculate the average acoustic attenuation coefficient relative to the inner tissue of the suspect area ($A_j$), considering that the whole acoustic attenuation calculated for the $i^{th}$ line ($A_i$) is given by the sum of the acoustic attenuation along the healthy portions (of Li' and Li" length) and of the acoustic attenuation along the portion contained inside the suspect area (of Li‴ length), according to the following relation, in which the only unknown factor is the value of the average acoustic attenuation coefficient relative to the inner tissue of the suspect area ($A_j$), since the average acoustic attenuation coefficient along the whole $i^{th}$ propagation line ($A_i$) can be calculated considering the propagation line as it would be crossing an homogeneous tissue:

$$Ats \times (Li' + Li'')/(Li) + Aj \times Li'''/Li = Ai$$

It is to be précised that the measurement of the lengths of the outer and inner segment of the suspect area (Li', Li'', Li''') is made possible only by the acquisition of an ultrasound image carried out in the same position of acquisition, and with a frequency greater than the ultrasound signal, in order to improve the resolution of the ultrasound image, and so the precision of the calculation of the lengths of the outer and inner segment of the suspect area (Li', Li'', Li''').

In other words, this measurement is made possible only by the configuration of the arrays of piezoelectric transducers of the device according to the invention, which comprises two ultrasound probes opposite to each other, each one comprising two arrays with different nominal frequencies, the one optimized for the acquisition of a high resolution ultrasound image and the other one for the calculation of the acoustic attenuation coefficient, and generally for the execution of acoustic measurements in transmission at lower frequencies than the ones needed for obtaining good ultrasound images. In a preferred embodiment, for the calculation of the diagnostic parameter, the method can be carried out according to the following steps.

210) calculation of a plurality of morphological and intensity characteristics deriving from the ultrasound imaging of the $j^{th}$ area (Zj), among which for example:

- volume of the area;
- surface to volume ratio;
- maximum dimension;
- parameters of shape, such as eccentricity;
- ratio between the average grey value of the $j^{th}$ area (Zj) and the average grey value of the surrounding region.

Preferably, said surrounding region is defined as the assembly of all the portions of breast tissue not belonging to any suspect area or as the assembly of all the portions of breast tissue not belonging to any suspect area and crossed by the same propagation lines of the considered $j^{th}$ area (Zj).

220) calculation of the average acoustic attenuation coefficient (Aj) relative to at least a portion of propagation line of the ultrasound signal contained inside the $j^{th}$ area (Zj);
230) calculation of the ratio between the average acoustic attenuation coefficient (Aj) of the $j^{th}$ area (Zj) and average acoustic attenuation coefficient of the outer region to the $j^{th}$ area (Ats),
235) calculation of the acoustic propagation speed of the ultrasound signal relating to at least a propagation line of the ultrasound signal passing through the $j^{th}$ area (Zj) and at least a propagation line of the ultrasound signal not passing through any suspect area.

Conveniently, the propagation speed, which gives an indication of the tissue density, can be calculated as a function of the receiving time of the ultrasound signal transmitted and of the relative distance between the probes (both measured automatically by the device according to the present invention).

240) calculation of the BUB (Broadband Ultrasound Backscatter) relative to the assembly of the 3d model points inside the $j^{th}$ area (Zj);
It is to be specified that the BUB is the average of the backscattering coefficient (which is a parameter depending on the frequency) in a determined frequency interval, preferably between 0.2 and 0.6 MHz or anyway in a frequency interval in which the spectrum of the reflected signal has a decreasing development. The backscattering coefficient can be calculated as the ratio between the intensity of the ultrasound signal reflected by the portion of tissue considered and a reference ultrasound signal. The signal reflected by the air-water interface positioned at the same distance from the probe with respect to the portion of tissue in the examination step can be considered as reference ultrasound signal. So, it is clear that the reference ultrasound signal is a parameter characterizing the probe once the parameters of the transmitted signal (frequency, power, etc.) and the reference distance are fixed.
250) calculation of the IRC (Integrated Reflection Coefficient) relative to the interface between the $j^{th}$ area (Zj) and the surrounding tissue.
IRC can be calculated as the average of the energy reflection coefficient in a determined frequency interval, preferably in the interval between 0.2 and 0.6 MHz, or anyway in a frequency interval in which the spectrum of the reflected signal has a decreasing development, and is obtained as logarithmic difference between the spectrum of the signal reflected by said interface in the analysis step (between the $j^{th}$ area and the surrounding tissue) and the signal reflected by the air-water reference interface (stored in the system in the construction step of the device and available for the calculation). Downwards of point 250), so it is defined a set of values of each parameter relative to each ozone (Zj).

260) calculation of at least a frequency spectrum of the radiofrequency raw ultrasound signal emitted by one of said first arrays (11, 21) and reflected by a segment of a propagation line of the ultrasound signal contained inside the $j^{th}$ area.

270) calculation of at least a quantitative parameter extracted by the spectrum of point 260), included in the list comprising the following parameters:

- spectrum average value;
- area subtended by the spectrum in a determined frequency interval;
- spectrum width (intended as the difference between maximum and minimum frequency) at a predetermined intensity level, in particular at a level defined by an intensity value lower than the maximum value of said spectrum for a predetermined amount, in particular lower than 1 dB or 3 dB;
- the frequency value corresponding to the maximum value of said spectrum;
- the slope of a line interpolating a plurality of points of said spectrum in a predetermined frequency interval;
- the coefficients of a polynomial interpolating the points of said frequency spectrum in a frequency interval containing the maximum of said spectrum.

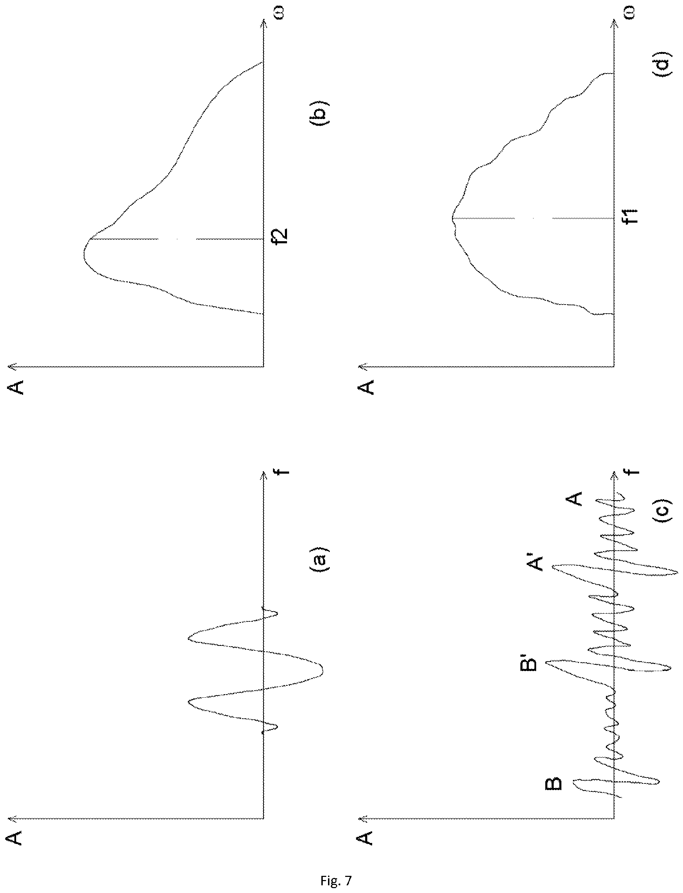

Figure 6 shows the portion (110') of the propagation line (110) of the ultrasound signal emitted by a piezoelectric crystal of the first array (11) of the first probe (10) comprised inside the $j^{th}$ area (Zj). Figure 7 shows the relative ultrasound signal acquired in reflection in the time domain, and in particular the portion (A'-B') associated to the portion of the propagation line contained inside the $j^{th}$ area (Zj). Figure 7 shows also the spectrum (S1) obtained as frequency transform of the ultrasound signal reflected by the tissue comprised inside the $j^{th}$ area (Zj).

Preferably, the frequency spectrum associated to the $j^{th}$ area (Zj) is calculated as the average spectrum of a plurality of spectra associated to a plurality of propagation segments of the signal contained inside the $j^{th}$ area. Preferably, for the calculation of the average spectrum a selection step of the ultrasound signals is also carried out, to be used for the calculation of the average spectrum relative to the $j^{th}$ area;

- calculation of the correlation coefficient of the average spectrum with all the spectra used for its calculation;
- exclusion of the spectra for which such correlation coefficient is lower than a predetermined threshold, for example 0.9;
- calculation of a new average spectrum relative to the $j^{th}$ area;
- repetition of the calculation procedure of the spectra correlation and exclusion, until all the spectra left have a correlation coefficient with the average spectrum greater than or equal to said predetermined threshold.

280) Calculation of a diagnostic parameter which is a function of the comparison of at least one of the parameters calculated in steps 210, 220, 230, 235, 240, 250, 260, 270, with

- the respective parameters relating to tumors detected in ultrasound examinations carried out on patients for whom the presence of a breast cancer has been subsequently confirmed by means of histologic examination;
- the respective parameters relating to suspect tumors detected in ultrasound examinations carried out on patients for whom the presence of a breast cancer has been subsequently excluded by means of a biopsy or other equivalent reliable technique;
- the respective parameters relative to portions of tissue not interested by cancers, detected in ultrasound examinations carried out on patients for whom the presence of a breast cancer has been subsequently excluded.

So, it is clear that the comparison can occur only after execution of examinations by means of the method according to the invention on a statistically significant number on patients, for whom the possible presence of a tumor has been subsequently confirmed by other tests, and that a data set is then available, acquired according to what just described and relative to a set of suspect areas (Zt), whose tumoral nature has been confirmed, to a set of suspect areas (Znt), whose tumoral nature has been excluded, and to a set of portions of tissues not interested by ultrasound visible suspect areas (Tnz).

Therefore, the comparison occurs downwards of the following procedure for reference values definition for the parameters.

300) Definition, for each parameter calculated at points 210 to 250, of an average tumoral value (VMt), and a relative confidence interval at 75% and 95%, calculated starting from the statistic distribution of the values of each parameter for all the suspect areas belonging to said set of suspect areas (Zt) whose tumoral nature has been confirmed;

310) definition, for each of the parameters calculated at points 210 to 250, of a not tumoral average value (VMnt), and a relative confidence interval at 75% and 95%, calculated starting from the statistical distribution of the values of each parameter for all the suspect areas belonging to said set of suspect areas, whose tumoral nature (Znt) has been excluded;

320) definition, for each parameter calculated at points 210 to 250, of a healthy average value (VMs), and a relative confidence interval at 75% and 95%, calculated starting from the statistic distribution of the values of each parameter for all the portions of tissues belonging to said set of portions of tissues not interested by ultrasound visible suspect areas (Tnz).

Downwards of point 320), three reference sets of values of each parameter are then defined: a first set relative to tumoral areas, a second set relative to suspect areas, which then result not tumoral, a third set relative to a tissue not belonging to suspect areas (healthy tissue).

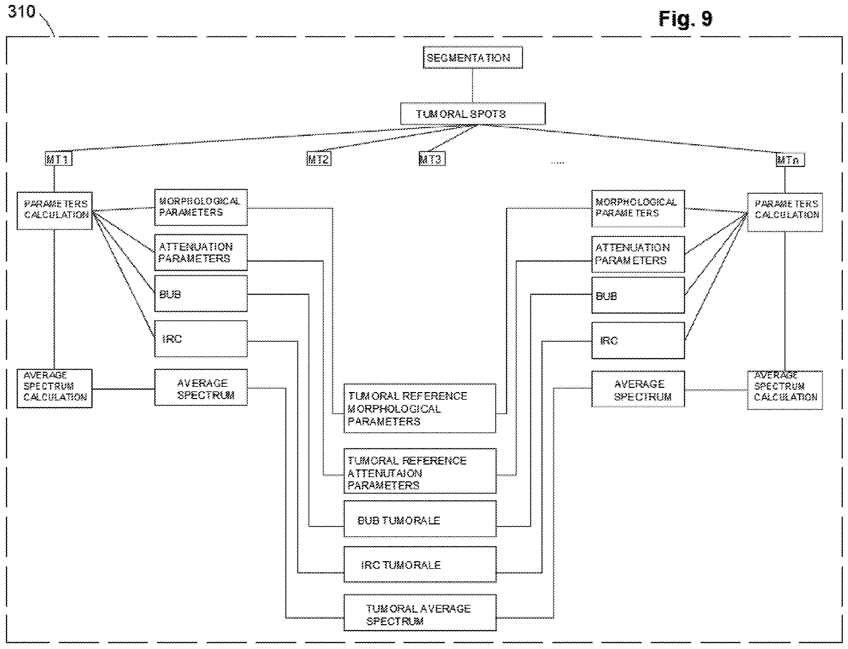

With reference to the calculation of the tumoral reference values, the step 310) is shown in the flowchart of figure 9, where it is observed as for all the tumoral spots (XT1,... ZTn) all the parameters (morphological, of acoustic attenuation, relative to BUB and IRC and relative to frequency spectra) are calculated, and for each parameter is then calculated the average of all the values calculated for the single tumoral areas to obtain a set of tumoral reference values comprising an average value for each parameter.

The procedure is the same for the not tumoral suspect areas and for the tissue interested by ultrasound not visible suspect areas.

The method can possibly provide the following selection step of significant parameters.

330) selection among the parameters of the reference sets of the ones for which the average tumoral value (VMt) is outside both the confidence interval at 75% of the relative healthy average value (VMs) and the confidence interval at 75% of the relative not tumoral average value (VMnt).

The definition procedure of the reference values comprises also the calculation of a series of reference frequency spectra.

340) selection, from the data acquired in ultrasound examinations carried out on patients for whom the breast cancer presence/absence diagnosis has been confirmed, of a plurality of segments of ultrasound propagation:

- contained inside tumoral areas;
- contained inside suspect areas, subsequently resulted not tumoral;
- contained inside the outer tissue of the suspect areas;

350) calculation of the frequency transform of the radiofrequency raw ultrasound signal associated to each of said segments of ultrasound propagation;
360) calculation:

- of the tumoral reference spectrum obtained as the average spectrum relative to all the ultrasound signals relating to tumoral areas;
- of the not tumoral reference spectrum obtained as the average spectrum relative to all the ultrasound signals relating to not tumoral areas;
- of the reference spectrum relative to the outer tissues of the suspect areas obtained as the average spectrum relative to all the ultrasound signals relating to the outer tissue of the suspect areas.

Moreover, preferably the method comprises a selection step of the ultrasound signals to be used for the calculation of the reference spectra, defined as follows:
370) for each of the reference spectra calculated at point 360:

- calculation of the correlation coefficient of the reference spectrum with all the spectra used for its calculation;
- exclusion of the spectra whose correlation coefficient is lower than a predetermined threshold, for example 0.9;
- calculation of a new reference spectrum;
- repetition of the calculation procedure of the spectra correlation and exclusion, until all the spectra left have a correlation coefficient with the reference spectrum greater than or equal to said predetermined threshold.

Once the reference values of the parameters and the value of the reference frequency spectrum are known, a diagnostic parameter indicating the probability that the $j^{th}$ area (Zj) is tumoral or not is calculated.

In a first embodiment, said diagnostic parameter is a classification of the $j^{th}$ suspect area (Zj) as tumoral or not tumoral. In order to classify the $j^{th}$ area, the procedure is the following;

400) calculation of the correlation coefficient of the set of parameters relative to the $j^{th}$ area,

- with the reference set relative to tumoral areas,
- with the reference set relative to suspect areas, subsequently resulted not tumoral;

- with the reference set relative to tissue not belonging to suspect areas.

410) In case one of the three coefficients calculated at point 400 is greater than a first predetermined threshold (for example r > 0.9) and the remaining ones are lower than a second predetermined threshold (for example r < 0.7), classification of the $j^{th}$ area as tumoral if the correlation coefficient is greater than the one with the reference set relative to tumoral area, as not tumoral if it is one of the other two;
420) in case no one of the correlation coefficients is greater than said first predetermined threshold, or in case the two lower correlation coefficients are both not lower than said second predetermined threshold, the $j^{th}$ area is not classified.

In another embodiment, for the classification of the $j^{th}$ area, the procedure is the following:

500) calculation of the correlation coefficient of the average spectrum relative to the $j^{th}$ area with

- the reference spectrum of a tumoral area;
- the reference spectrum of a suspect area, subsequently resulted not tumoral;
- the reference spectrum relative to the outer tissues of the suspect areas.

510) In case one of the three coefficients calculated at point 500 is greater than a first predetermined threshold (for example r > 0.9) and the remaining ones are lower than a second predetermined threshold (for example r < 0.7), classification of the $j^{th}$ area as tumoral if the correlation coefficient is greater than the one with the tumoral reference spectrum, as not tumoral if it is one of the other two;
520) in case no one of the correlation coefficients is greater than said first predetermined threshold, or in case the two lower correlation coefficients are both not lower than said second predetermined threshold, the $j^{th}$ area is not classified.

In another embodiment, for the classification of the $j^{th}$ area, the procedure is the following:

600) subdivision of the reference data sets available in a training data set and a validation data set,
610) training of a classification neural network, by using said training set, to classify a data set relative to a tumoral or not tumoral suspect area, 620) presentation of the data set relative to the $j^{th}$ area to the trained network,
630) classification of the $j^{th}$ area as tumoral or not tumoral as a function of the network output.

In a second embodiment, said diagnostic parameter is a numerical value of the probability that said $j^{th}$ suspect area (Zj) is tumoral or not tumoral.
Said numerical value of the probability that the $j^{th}$ suspect area (Zj) is tumoral or not can be calculated by converting in percentage value said correlation coefficient of the average spectrum relative to the $j^{th}$ area with the reference spectrum of a tumoral area calculated at point 500).

## Claims

1.  Ultrasound device (1) for carrying out a breast diagnostic examination for breast cancer diagnosis comprising two ultrasound probes (10, 20),
    **characterized in that**

    each of said probes (10, 20) comprises a first array (11, 21) of piezoelectric or CMUT transducers with a first nominal frequency (f1), and a second array of piezoelectric or CMUT transducers (12, 22) with a second nominal frequency (f2), said probes (10, 20) being arranged opposite to each other, being configured to rotate along a circular trajectory having for centre the midpoint of the segment connecting them and to slide to each other along said straight line connecting them, so that they come in contact with the breast of the patient from diametrically opposite portions,
    **in that**
    said arrays (11, 12, 21, 22) comprise each a plurality of piezoelectric or CMUT transducers, positioned so that acquisitions are carried out on a plane of acquisition orthogonal to the plane of said circular trajectory,
    and **in that**
    said device is configured to carry out, by moving said probes (10, 20) along said circular trajectory, a plurality of scans relative to a plurality of planes of acquisition (P0, P1...) passing through said midpoint, orthogonal to said circular trajectory and rotated to each other of an angle ($\alpha$).

2. Ultrasound device (1) for carrying out a breast diagnostic examination for breast cancer diagnosis, according to claim 1, **characterized in that**

said device comprises also a circular support (30), and **in that**
each probe (10, 20) comprises also a fixing rod (13, 23) associated to said circular support (30), so that the two probes are positioned along the same direction, opposite to each other, with the arrays (11, 12, 21, 22) positioned in the same plane of the axis of symmetry (a) of said circular guide (30).

3. Ultrasound device (1) for carrying out a breast diagnostic examination for breast cancer diagnosis, according to claim 1 or 2, **characterized in that** the shape of the head of each of said probes (10, 20) is concave, so that a better breast shape adherence is allowed.

4. Ultrasound device (1) for carrying out a breast diagnostic examination for breast cancer diagnosis, according to any one of the preceding claims, **characterized in that** said first nominal frequency (f1) is chosen to carry out a B-Mode ultrasound imaging starting from the signals reflected by the breast tissues,
and said second nominal frequency (f2) is chosen to carry out acoustic attenuation measures, working in transmission mode with the array of the other probe having the same nominal frequency.

5. Ultrasound device (1) for carrying out a breast diagnostic examination for breast cancer diagnosis, according to any one of the preceding claims, **characterized in that** said first nominal frequency (f1) is between 7 and 10 MHz and said second nominal frequency (f2) is between 1 and 3 MHz.

6. Ultrasound device (1) for carrying out a breast diagnostic examination for breast cancer diagnosis, according to any one of the preceding claims, **characterized in that** each probe comprises also acoustic coupling means (14, 15) with the breast skin, comprising a flexible membrane (14), filled with a gel (15), the assembly of membrane (14) and gel (15) being configured to be pressed when the probe is thrusted towards the breast, thus adapting to the breast shape and thus guaranteeing mechanic coupling for ultrasound transmission.

7. Ultrasound device (1) for carrying out a breast diagnostic examination for breast cancer diagnosis, according to any one of the preceding claims, further comprising electronic computing means on which computer programs are loaded configured to carry out, for each position of acquisition, the following procedure of acquisition:

   100) acquisition of a first B-mode ultrasound image relating to the portion of breast tissues comprised between said first probe (10) and said axis of symmetry, by means of said first array (11) of said first probe (10);
   110) acquisition of a second B-mode ultrasound image relating to the portion of tissues comprised between said second probe (10) and said axis of symmetry, by means of said first array (21) of said second probe (20);
   120) storage of "raw" reflected ultrasound signals, as detected by each probe (10, 20) before any further processing, in particular before the processing for the corresponding B-mode image creation;
   130) emission of an ultrasound pulse by means of the first piezoelectric or CMUT transducer (121) of said second array (12) of said first probe (10), and detection:

   - of the relative ultrasound signal transmitted by means of the first piezoelectric or CMUT transducer (221) of said second array (22) of said second probe (20) and
   - of the relative ultrasound signal reflected by means of said first piezoelectric or CMUT transducer (121) of said second array (12) of said first probe (10),

   140) repetition of step 130) for each couple of piezoelectric or CMUT transducers of said second arrays (12, 22) of said first (10) and second probe.

8. Ultrasound device (1) for carrying out a breast diagnostic examination for breast cancer diagnosis, according to claim 7, **characterized in that** it is configured to carry out a plurality of acquisitions in a series of acquisition planes (P0, P1), rotated to each other of a predetermined angle ($\alpha$), so that to each point of each plane of acquisition is associated:

   - a value ($S_i$) relating to the intensity of the reflected ultrasound signal,
   - a value ($B_i$) of the grayscale, associated to the B-mode ultrasound image, the group of said values ($B_i$) thus

constituting a 3d ultrasound model of the breast volume,

and **in that**
for each position of acquisition are stored:

- raw radiofrequency reflected ultrasound signals relating to all the propagation lines of the ultrasound signal of each first (11, 21) and second array (12, 22),
- raw radiofrequency transmitted ultrasound signals, relating to all the propagation lines of the ultrasound signal of said second arrays (12, 22) .

9. Ultrasound device (1) for carrying out a breast diagnostic examination for breast cancer diagnosis, according to claim 8, **characterized in that** said computer programs are configured to carry out a method for calculating a diagnostic parameter representing the presence or not of a breast cancer comprising the steps of:

200) segmentation of said 3d ultrasound model of the breast volume to individuate the presence of one or more suspect areas (Zj), **characterized by** brighter colour (hyperechoic) or darker colour (hypoechoic) with respect to the surrounding tissue, or anyway **characterized by** any other inhomogeneity detectable by means of grayscale analysis,
201) calculation of a diagnostic parameter (Dj) referred to each suspect area (Zj), indicating the probability that such area can be classified as breast cancer, said diagnostic parameter (Dj) depending on one or more of the following factors:

(i) morphological information deriving from the B-mode imaging of the points of said 3D model inside the $i^{th}$ area (Zj);
(ii) information relating to the average acoustic attenuation coefficient relative to at least a portion of propagation line of the ultrasound signal contained inside the $j^{th}$ area (Zj);
(iii) information relating to the frequency spectrum of the signal associated to the $j^{th}$ area (Zj).

10. Ultrasound device (1) for carrying out a breast diagnostic examination for breast cancer diagnosis, according to claim 9, **characterized in that** said diagnostic parameter is calculated by means of the method according to the following steps of:

210) calculation of a plurality of morphological characteristics deriving from the ultrasound imaging of the $j^{th}$ area (Zj), chosen from the list below:

- volume of the area;
- surface to volume ratio;
- maximum dimension;
- eccentricity;
- ratio between the average grey value of the jth area (Zj) and the average grey value of the surrounding region;

220) calculation of the average acoustic attenuation coefficient (Aj) relative to at least a portion of propagation line of the ultrasound signal contained inside the $j^{th}$ area (Zj) ;
230) calculation of the ratio between said average acoustic attenuation coefficient and the average acoustic attenuation coefficient of the outer region to the $j^{th}$ area (Ats);
235) calculation of the acoustic propagation speed of the ultrasound signal relating to at least one propagation line of the ultrasound signal passing through the $j^{th}$ area (Zj) and to at least one propagation line of the ultrasound signal not passing through any suspect area;
260) calculation of at least one frequency spectrum of the radiofrequency raw ultrasound signal emitted by one of said first arrays (11, 21) and reflected by a tissue portion corresponding to a segment of a propagation line of the ultrasound signal contained inside the $j^{th}$ area;
270) calculation of at least one quantitative parameter extracted by the spectrum of point 260), chosen from the list comprising the following parameters:

- spectrum average value;
- area subtended by the spectrum in a determined frequency interval and/or determined intensity interval;
- spectrum width in a predetermined frequency interval;

- frequency value corresponding to the maximum value of said spectrum;
- slope of a line interpolating a plurality of points of said spectrum in a predetermined frequency interval;
- coefficients of a polynomial interpolating the points of said spectrum in a frequency interval containing the maximum of said spectrum;

280) calculation of a diagnostic parameter function of the comparison of at least one of the parameters calculated in steps 210 to 270 with respective parameters obtained by means of ultrasound examinations carried out by means of the device according to any one of the preceding claims and referred to:

- suspect areas for which the presence of the pathology has been subsequently confirmed by means of histologic examination;
- suspect areas for which the presence of a breast cancer has been subsequently excluded by means of a biopsy or other equivalent reliable technique;
- portions of tissue not interested by suspect areas.

11. Ultrasound device (1) for carrying out a breast diagnostic examination for breast cancer diagnosis, according to claim 10, **characterized in that** at point 260) a frequency spectrum is calculated associated to the $j^{th}$ area (Zj) as the average of a plurality of spectra relative to respective propagation segments of the signal contained inside said suspect area (Zj),
and **in that**
for the calculation of the average spectrum the following spectra selection procedure is carried out, to be used for the calculation of the average spectrum relative to the $j^{th}$ area:

- calculation of the correlation coefficient of the average spectrum with all the spectra used for its calculation;
- exclusion of the spectra for which such correlation coefficient is lower than a predetermined threshold;
- calculation of a new average spectrum relative to the $j^{th}$ area;
- repetition of the calculation procedure of the spectra correlation and exclusion, until all the spectra left have a correlation coefficient with the average spectrum greater than or equal to said predetermined threshold.

12. Ultrasound device (1) for carrying out a breast diagnostic examination for breast cancer diagnosis, according to claim 11, **characterized in that** said comparison of point 280) occurs downwards of the following definition procedure of tumoral, not tumoral and healthy reference value sets, detected in ultrasound examinations carried out by means of the device according to any one of the preceding claims on patients for whom the presence of the tumoral pathology has been subsequently confirmed or excluded by means of histologic examination:

300) definition, for each parameter calculated at points 210 to 250, of an average tumoral value (VMt), and of a relative confidence interval at 75% and 95%, calculated starting from the statistic distribution of the values of each parameter for all the suspect areas whose tumoral nature has been confirmed;
310) definition, for each of the parameters calculated at points 210 to 250, of a not tumoral average value (VMnt), and of a relative confidence interval at 75% and 95%, calculated starting from the statistical distribution of the values of each parameter for all the suspect areas whose tumoral nature (Znt) has been excluded;
320) definition, for each parameter calculated at points 210 to 250, of a healthy average value (VMs), and of a relative confidence interval at 75% and 95%, calculated starting from the statistic distribution of the values of each parameter for all the portions of tissues belonging to said set of portions of healthy tissue not interested by ultrasound-visible suspect areas (Tnz);
340) selection of a plurality of segments of propagation of the ultrasound signal:

- contained inside tumoral areas;
- contained inside suspect areas, subsequently resulted not tumoral;
- contained inside tissue located outside of suspect areas;

350) calculation of the frequency transform of the radiofrequency raw ultrasound signal associated to each of said segments of propagation of the ultrasound signal;
360) calculation:

- of the tumoral reference spectrum obtained as the average spectrum relative to all the ultrasound signals relating to tumoral areas;
- of the not tumoral reference spectrum obtained as the average spectrum relative to all the ultrasound signals relating to suspect areas, subsequently resulted not tumoral;
- of the reference spectrum relative to the outer tissues of the suspect areas obtained as the average spectrum relative to all the ultrasound signals relating to the outer tissue of the suspect areas.

13. Ultrasound device (1) for carrying out a breast diagnostic examination for breast cancer diagnosis, according to claim 10, **characterized in that** said definition procedure of tumoral reference value sets comprises the step of:
330) selection of the parameters for which the average tumoral value (VMt) is outside both the confidence interval at 75% of the relative healthy average value (VMs), and the confidence interval at 75% of the relative not tumoral average value (VMnt).

14. Ultrasound device (1) for carrying out a breast diagnostic examination for breast cancer diagnosis, according to claim 10, **characterized in that** said diagnostic parameter is a classification of said $j^{th}$ suspect area (Zj) as "tumoral" or "not tumoral", obtained by:

400) calculation of the correlation coefficient of the set of parameters relative to the $j^{th}$ area

- with the reference set relative to tumoral areas,
- with the reference set relative to suspect areas, resulted not tumoral in subsequent analyses;
- with the reference set relative to tissue not belonging to suspect areas,

410) in case one of the three coefficients calculated at point 400) is greater than a first predetermined threshold and the remaining ones are lower than a second predetermined threshold, classification of the $j^{th}$ area as tumoral if the correlation coefficient is greater than the one with the reference set relative to tumoral areas, as not tumoral if it is one of the other two;
420) in case no one of the correlation coefficients is greater than said first predetermined threshold, or in case the two lower correlation coefficients are both not lower than said second predetermined threshold, the $j^{th}$ area is not classified and further diagnostic examinations are recommended.

15. Ultrasound device (1) for carrying out a breast diagnostic examination for breast cancer diagnosis, according to claim 10, **characterized in that** said diagnostic parameter is a classification of the $j^{th}$ suspect area (Zj) as tumoral or not tumoral, obtained by:

500) calculation of the correlation coefficient of the average spectrum relative to the $j^{th}$ area with

- the tumoral reference spectrum;
- the reference spectrum of a suspect area, subsequently resulted not tumoral;
- the reference spectrum relative to the outer tissues of the suspect areas calculated at point 360);

510) in case one of the three coefficients calculated at point 500 is greater than a first predetermined threshold and the remaining ones are lower than a second predetermined threshold, classification of the $j^{th}$ area as tumoral if the correlation coefficient is greater than the one with the tumoral reference spectrum, as not tumoral if it is one of the other two;
520) in case no one of the correlation coefficients is greater than said first predetermined threshold, or in case the two lower correlation coefficients are both not lower than said second predetermined threshold, the $j^{th}$ area is not classified.

16. Ultrasound device (1) for carrying out a breast diagnostic examination for breast cancer diagnosis, according to claim 10, **characterized in that** said diagnostic parameter is a classification of the $j^{th}$ suspect area as tumoral or not tumoral, obtained by:

600) subdivision of the reference data sets available in a training data set and a validation data set,
610) training of a classification neural network, by using said training set, to classify a data set relative to a tumoral or not tumoral suspect area,
620) presentation of the data set relative to the $j^{th}$ area to the trained network,
630) classification of the $j^{th}$ area as tumoral or not tumoral as a function of the network output.

17. Ultrasound device (1) for carrying out a breast diagnostic examination for breast cancer diagnosis, according to claim 15, **characterized in that** said diagnostic parameter is a numerical value of the probability that said $j^{th}$ suspect area (Zj) is tumoral or not tumoral, calculated by converting in percentage value said correlation coefficient of the average spectrum relative to the jth area with the reference spectrum of a tumoral area calculated at point 500).

**Patentansprüche**

1. Ultraschallvorrichtung (1) zur Durchführung einer diagnostischen Untersuchung der Brust zur Diagnose von Brustkrebs umfassend zwei Ultraschallsonden (10, 20),

   **dadurch gekennzeichnet, dass** jede der Sonden (10, 20) eine erste Anordnung (11, 21) piezoelektrischer Wandler oder CMUT mit einer ersten Nennfrequenz (f1) und eine zweite Anordnung piezoelektrischer Wandler oder CMUT (12, 22) mit einer zweiten Nennfrequenz (f2) umfasst, wobei die Sonden (10, 20) einander gegenüber angeordnet und so konfiguriert sind, dass sie sich entlang einer kreisförmigen Bahn drehen, deren Mittelpunkt die Mitte des sie verbindenden Segments ist, und dass sie entlang der sie verbindenden geraden Linie aufeinander zu gleiten, so dass sie durch diametral gegenüberliegende Teile mit der Brust der Patientin in Kontakt kommen, und
   dass die Anordnungen (11, 12, 21, 22) jeweils mehrere piezoelektrische Wandler oder CMUTs umfassen, die so positioniert sind, dass die Erfassungen auf einer Erfassungsebene orthogonal zur Ebene der kreisförmigen Trajektorie durchgeführt werden,
   und dass die Vorrichtung so konfiguriert ist, dass sie durch Bewegen der Sonden (10, 20) entlang der kreisförmigen Trajektorie mehrere Scans in Bezug auf mehrere Erfassungsebenen (P0, P1...) durchführt, die orthogonal durch den Mittelpunkt der besagten Kreisbahn verlaufen und relativ zueinander um einen Winkel ($\alpha$) verdreht werden.

2. Ultraschallvorrichtung (1) zur Durchführung einer diagnostischen Untersuchung der Brust zur Diagnose von Brustkrebs, nach Anspruch 1, **dadurch gekennzeichnet,**

   **dass** die Vorrichtung außerdem einen kreisförmigen Träger (30) umfasst, und
   **dass** jede Sonde (10, 20) außerdem eine Befestigungsstange (13, 23) umfasst, die mit dem kreisförmigen Träger (30) verbunden ist, so dass die beiden Sonden in der gleichen Richtung, einander gegenüber, mit den Anordnungen (11, 12, 21, 22), in derselben Ebene der Symmetrieachse (a) der kreisförmigen Führung (30) positioniert sind.

3. Ultraschallvorrichtung (1) zur Durchführung einer diagnostischen Untersuchung der Brust zur Diagnose von Brustkrebs, nach Anspruch 1 oder 2, **dadurch gekennzeichnet, dass** die Kopfform jeder der Sonden (10, 20) konkav ist, so dass eine bessere Anpassung an die Brustform ermöglicht wird.

4. Ultraschallvorrichtung (1) zur Durchführung einer diagnostischen Untersuchung der Brust zur Diagnose von Brustkrebs, nach einem der vorhergehenden Ansprüche, **dadurch gekennzeichnet, dass** die erste Nennfrequenz (f1) zur Durchführung der Bildgebung bei B-Mode-Ultraschall ausgehend von Signalen, die vom Brustgewebe reflektiert werden, gewählt wird, und
   dass die zweite Nennfrequenz (f2) zur Durchführung akustischer Dämpfungsmessungen gewählt wird, wobei im Übertragungsmodus mit der Anordnung der anderen Sonde mit derselben Nennfrequenz gearbeitet wird.

5. Ultraschallvorrichtung (1) zur Durchführung einer diagnostischen Untersuchung der Brust zur Diagnose von Brustkrebs, nach einem der vorhergehenden Ansprüche, **dadurch gekennzeichnet, dass** die erste Nennfrequenz (f1) zwischen 7 und 10 MHz liegt und die zweite Nennfrequenz (f2) zwischen 1 und 3 MHz liegt.

6. Ultraschallvorrichtung (1) zur Durchführung einer diagnostischen Untersuchung der Brust zur Diagnose von Brustkrebs, nach einem der vorhergehenden Ansprüche, **dadurch gekennzeichnet, dass** jede Sonde außerdem akustische Kopplungsmittel (14, 15) mit der Haut der Brust aufweist, umfassend eine flexible Membran (14), die gefüllt mit einem Gel (15) ist, wobei die Anordnung aus Membran (14) und Gel (15) so konfiguriert ist, dass sie gedrückt wird, wenn die Sonde in Richtung der Brust gedrückt wird, und sich so an die Form der Brust anpasst und so eine mechanische Ankopplung für die Ultraschallübertragung gewährleistet.

7. Ultraschallvorrichtung (1) zur Durchführung einer diagnostischen Untersuchung der Brust zur Diagnose von Brustkrebs, nach einem der vorangehenden Ansprüche, weiterhin elektronische Berechnungsmittel umfassend, auf denen Computerprogramme geladen sind, die für die Durchführung des folgenden Erwerbsverfahrens für jede Erwerbsposition jeweils konfiguriert sind

100) Erfassung eines ersten B-Mode-Ultraschallbildes, das sich auf den Teil des Brustgewebes zwischen der ersten Sonde (10) und der Symmetrieachse bezieht, mittels der ersten Anordnung (11) der ersten Sonde (10);
110) Erfassung eines zweiten B-Mode-Ultraschallbildes bezüglich des Gewebeteils zwischen der zweiten Sonde (10) und der Symmetrieachse mittels der ersten Anordnung (21) der zweiten Sonde (20);
120) Speichern der "rohen" reflektierten Ultraschallsignale, wie sie von jeder Sonde (10, 20) erfasst wurden, vor jeder nachfolgenden Verarbeitung, insbesondere vor der entsprechenden B-Modus-Bilderzeugungsverarbeitung;
130) Aussenden eines Ultraschallimpulses mittels des ersten piezoelektrischen Wandlers oder CMUT (121) der zweiten Anordnung (12) der ersten Sonde (10) und Detektion:

- des relativen Ultraschallsignals, das mittels des ersten piezoelektrischen Wandlers oder CMUT (221) der zweiten Anordnung (22) der zweiten Sonde (20) übertragen wird, und
- des relativen Ultraschallsignals, das mittels des ersten piezoelektrischen Wandlers oder CMUT (121) der zweiten Anordnung (12) der ersten Sonde (10) reflektiert wird,

140) Wiederholung von Schritt 130) für jedes Paar piezoelektrischer Wandler oder CMUTs der zweiten Anordnungen (12, 22) der ersten (10) und zweiten Sonden.

8. Ultraschallvorrichtung (1) zur Durchführung einer diagnostischen Untersuchung der Brust zur Diagnose von Brustkrebs, nach Anspruch 7, **dadurch gekennzeichnet, dass** es zur Durchführung mehrerer Aufnahmen in einer Reihe von Aufnahmeebenen (P0, P1) konfiguriert ist, die relativ zueinander um einen vordefinierten Winkel ($\alpha$) gedreht sind, sodass jedem Punkt jeder Erfassungsebene Folgendes zugeordnet ist:

- ein Wert (Si), der sich auf die Intensität des reflektierten Ultraschallsignals bezieht,
- ein Wert ($B_i$) der Grauskala, der dem Ultraschallbild im Modus B zugeordnet ist, wobei alle genannten Werte (Bi) somit ein 3D-Ultraschallmodell des Brustvolumens bilden, und für jede Positionserfassung gespeichert werden:
- Roh-Ultraschallsignale, die in Hochfrequenz reflektiert werden und sich auf alle Ausbreitungslinien des Ultraschallsignals jeder ersten (11, 21) und zweiten Anordnungen (12, 22) beziehen,
- per Hochfrequenz übertragene Roh-Ultraschallsignale, die sich auf alle Ultraschallsignalausbreitungslinien der zweiten Anordnungen (12, 22).

9. Ultraschallvorrichtung (1) zur Durchführung einer diagnostischen Untersuchung der Brust zur Diagnose von Brustkrebs, nach Anspruch 8, **dadurch gekennzeichnet, dass** die Computerprogramme zur Durchführung eines Verfahrens zur Berechnung eines für das Vorliegen oder Fehlen von Brustkrebs repräsentativen diagnostischen Parameters konfiguriert sind, einschließlich der folgenden Schritte:

200) Segmentierung des 3D-Ultraschallmodells des Brustvolumens, um das Vorhandensein einer oder mehrerer verdächtiger Zonen (Zj) zu individualisieren, die durch eine hellere (echoreiche) oder dunklere (echoarme) Farbe im Vergleich zum umgebenden Gewebe gekennzeichnet sind, oder auf jede andere Weise, die **dadurch gekennzeichnet ist, dass** sie jede andere mittels Graustufenanalyse erkennbare Inhomogenität umfasst,
201) Berechnung eines diagnostischen Parameters (Dj), der sich auf jeden verdächtigen Bereich (Zj) bezieht und die Wahrscheinlichkeit angibt, dass ein solcher Bereich als Brustkrebs klassifiziert werden kann, wobei dieser diagnostische Parameter (Dj) von einem oder mehreren der folgenden Faktoren abhängt:

(i) morphologische Informationen, die aus der B-Modus-Bildgebung der Punkte des 3D-Modells innerhalb der i-ten Zone (Zj) abgeleitet wurden;
(ii) Informationen über den durchschnittlichen akustischen Dämpfungskoeffizienten in Bezug auf mindestens einen Teil der Ausbreitungslinie des Ultraschallsignals, der in der j-ten Zone (Zj) enthalten ist;
(iii) Informationen über das Frequenzspektrum des Signals, das der j-ten Zone (Zj) zugeordnet ist.

10. Ultraschallvorrichtung (1) zur Durchführung einer diagnostischen Untersuchung der Brust zur Diagnose von Brustkrebs, nach Anspruch 9, **dadurch gekennzeichnet, dass** der genannte diagnostische Parameter mittels des Verfahrens gemäß den folgenden Schritten berechnet wird:

210) Berechnung einer Vielzahl morphologischer Merkmale aus der Ultraschallbildgebung der j-ten Zone (Zj), ausgewählt aus der folgenden Liste:

- Volumen der Fläche;
- Verhältnis Oberfläche/Volumen;
- maximale Abmessung;
- Exzentrizität;
- Verhältnis zwischen dem durchschnittlichen Grauwert der j-ten Zone (Zj) und dem durchschnittlichen Grauwert der umgebenden Region;

220) Berechnung des durchschnittlichen akustischen Dämpfungskoeffizienten (Aj), der sich auf mindestens einen Teil der Ausbreitungslinie des Ultraschallsignals bezieht, das innerhalb der j-ten Zone (Zj) enthalten ist;

230) Berechnung des Verhältnisses zwischen dem durchschnittlichen akustischen Dämpfungskoeffizienten und dem durchschnittlichen akustischen Dämpfungskoeffizienten des Bereichs außerhalb der j-ten Zone (Ats);

235) Berechnung der akustischen Ausbreitungsgeschwindigkeit des Ultraschallsignals in Bezug auf mindestens eine Ausbreitungslinie des Ultraschallsignals, die die j-te Zone (Zj) durchquert, und auf mindestens eine Ausbreitungslinie des Ultraschallsignals, die keine verdächtige Zone durchquert;

260) Berechnung mindestens eines Frequenzspektrums des rohen Hochfrequenz-Ultraschallsignals, das von einem der ersten Anordnungen (11, 21) ausgesendet und von einem Gewebeteil reflektiert wird, der einem Segment einer Ausbreitungslinie des Ultraschallsignalinhalts im Inneren der j-te Zone, entspricht;

270) Berechnung mindestens eines quantitativen Parameters, der aus dem Spektrum von Punkt 260 extrahiert wird, ausgewählt aus der Liste, die die folgenden Parameter umfasst:

- Durchschnittswert des Spektrums;
- vom Spektrum abgedeckter Bereich in einem bestimmten Frequenzintervall und/oder einem bestimmten Intensitätsintervall;
- Spektrumsbreite in einem vorgegebenen Frequenzintervall;
- Frequenzwert, der dem Maximalwert des Spektrums entspricht;
- Steigung einer Linie, die eine Vielzahl von Punkten des Spektrums in einem vorgegebenen Frequenzintervall interpoliert;
- Koeffizienten eines Polynoms, das die Punkte des Spektrums in einem Frequenzintervall interpoliert, das das Maximum des Spektrums enthält;

280) Berechnung eines diagnostischen Parameters basierend auf dem Vergleich mindestens eines der in den Schritten 210 bis 270 berechneten Parameter mit jeweiligen Parametern, die mittels Ultraschalluntersuchungen erhalten wurden, die mit der Vorrichtung nach einem der vorhergehenden Ansprüche durchgeführt wurden und auf die Bezug genommen wird:

- verdächtige Bereiche, bei denen das Vorliegen der Pathologie anschließend durch histologische Untersuchung bestätigt wurde;

280) Berechnung eines diagnostischen Parameters basierend auf dem Vergleich mindestens eines der in den Schritten 210 bis 270 berechneten Parameter mit jeweiligen Parametern, die mittels Ultraschalluntersuchungen erhalten wurden, die mit der Vorrichtung nach einem der vorhergehenden Ansprüche durchgeführt wurden und auf die Bezug genommen wird:

- verdächtige Bereiche, bei denen das Vorliegen der Pathologie anschließend durch histologische Untersuchung bestätigt wurde;
- verdächtige Bereiche, bei denen das Vorliegen von Brustkrebs anschließend mittels einer Biopsie oder einer anderen gleichwertigen zuverlässigen Technik ausgeschlossen wurde;
- Gewebeteile, die nicht von den verdächtigen Bereichen betroffen sind.

11. Ultraschallvorrichtung (1) zur Durchführung einer diagnostischen Untersuchung der Brust zur Diagnose von Brustkrebs, nach Anspruch 10, **dadurch gekennzeichnet, dass** im Punkt 260) ein der j-ten Zone (Zj) zugeordnetes Frequenzspektrum berechnet wird, das als Mittelwert einer Vielzahl von Spektren in Bezug auf die jeweiligen Ausbreitungssegmente des Signals, gilt, die in der verdächtigen Zone (Zj) enthalten sind, und dass für die Berechnung des Durchschnittsspektrums das folgende Spektrumauswahlverfahren durchgeführt wird, das für die Berechnung des Durchschnittsspektrums bezüglich der j-ten Zone, verwendet wird:

- Berechnung des Korrelationskoeffizienten des Durchschnittsspektrums mit allen für seine Berechnung verwendeten Spektren;
- Ausschluss von Spektren, für die ein solcher Korrelationskoeffizient kleiner als ein vorgegebener Schwellenwert ist;
- Berechnung eines neuen Durchschnittsspektrums in Bezug auf die j-te Zone;
- Wiederholung des Verfahrens zur Berechnung der Korrelation und des Ausschlusses der Spektren, bis alle verbleibenden Spektren einen Korrelationskoeffizienten mit dem Durchschnittsspektrum aufweisen, der größer oder gleich dem vorgegebenen Schwellenwert ist.

12. Ultraschallvorrichtung (1) zur Durchführung einer diagnostischen Untersuchung der Brust zur Diagnose von Brustkrebs, nach Anspruch 11, **dadurch gekennzeichnet, dass** der Vergleich von Punkt 280) im unteren Bereich des folgenden Definitionsverfahrens von Sätzen von Tumor-, Nicht-Tumor- und gesunden Referenzwerten, durchgeführt wird, die bei Ultraschalluntersuchungen mit der Vorrichtung nach einem der vorhergehenden Ansprüche bei Patienten ermittelt wurden, bei denen das Vorliegen der Tumorpathologie anschließend durch histologische Untersuchung bestätigt oder ausgeschlossen wurde:

300) Definition eines durchschnittlichen Tumorwerts (VMt) für jeden an den Punkten 210 bis 250 berechneten Parameter und eines Konfidenzintervalls relativ zu 75 % und 95 %, berechnet aus der statistischen Verteilung der Werte jedes Parameters für alle verdächtigen Bereiche, deren Tumornatur bestätigt wurde;
310) Definition eines mittleren Nichttumorwerts (VMnt) für jeden an den Punkten 210 bis 250 berechneten Parameter und eines Konfidenzintervalls relativ zu 75 % und 95 %, berechnet aus der statistischen Verteilung der Werte jedes einzelnen Parameter für alle verdächtigen Bereiche, aus denen die Tumornatur (Znt) ausgeschlossen wurde;
320) Definition eines gesunden Durchschnittswerts (VMs) für jeden an den Punkten 210 bis 250 berechneten Parameter und eines Konfidenzintervalls relativ zu 75 % und 95 %, berechnet aus der statistischen Verteilung der Werte jedes Parameters für alle Gewebeteile, die zu dem Satz gesunder Gewebeteile gehören und nicht von den verdächtigen Bereichen (Tnz) betroffen sind, die im Ultraschall sichtbar sind;
340) Auswahl mehrerer Ultraschallsignalausbreitungssegmente:

- Inhalte innerhalb der Tumorbereiche;
- Inhalte in verdächtigen Bereichen, die später keinen Tumor hervorgerufen haben;
- in Geweben außerhalb der verdächtigen Bereiche enthalten;

350) Berechnen der Frequenzumwandlung des rohen Ultraschallsignals in Hochfrequenz, die jedem der Ausbreitungssegmente des Ultraschallsignals zugeordnet ist;
360) Berechnung:

- des Referenz-Tumorspektrums, das als Durchschnittsspektrum aller Ultraschallsignale in Bezug auf die Tumorbereiche erhalten wird;
- des Nicht-Tumor-Referenzspektrums, das als Durchschnittsspektrum aller Ultraschallsignale in Bezug auf die verdächtigen Bereiche erhalten wird und anschließend zu Nicht-Tumor-Bereichen führt;
- des Referenzspektrums in Bezug auf das äußere Gewebe der verdächtigen Bereiche, das als Durchschnittsspektrum aller Ultraschallsignale in Bezug auf das äußere Gewebe der verdächtigen Bereiche erhalten wurde.

13. Ultraschallvorrichtung (1) zur Durchführung einer diagnostischen Untersuchung der Brust zur Diagnose von Brustkrebs, nach Anspruch 10, **dadurch gekennzeichnet, dass** das Verfahren zum Definieren von Referenztumorwertsätzen den Schritt umfasst:
330) Auswahl von Parametern, für die der mittlere Tumorwert (VMt) sowohl außerhalb des 75 %-Konfidenzintervalls des gesunden relativen Mittelwerts (VMs) als auch des 75 %-Konfidenzintervalls des mittleren relativen Nichttumorwerts (VMnt) liegt.

14. Ultraschallvorrichtung (1) zur Durchführung einer diagnostischen Untersuchung der Brust zur Diagnose von Brustkrebs, nach Anspruch 10, **dadurch gekennzeichnet, dass** der diagnostische Parameter eine Klassifizierung der j-ten verdächtigen Zone (Zj) als "Tumor" oder "Nicht-Tumor" ist, erhalten durch:

400) Berechnung des Korrelationskoeffizienten der Reihe von Parametern der j-ten Zone

- mit den Referenzreihen zu Tumorgebieten,
- mit der Referenzreihe, die sich auf verdächtige Bereiche bezieht, mit einem Nicht-Tumor-Ergebnis in nachfolgenden Analysen;
- mit der Referenzreihe zu Geweben, die nicht zu verdächtigen Bereichen gehören,

410) für den Fall, dass einer der drei bei Punkt 400) berechneten Koeffizienten größer als ein erster vorgegebener Schwellenwert ist und die anderen kleiner als ein zweiter vorgegebener Schwellenwert sind, Einstufung der j-ten Zone als Tumor, wenn der Korrelationskoeffizient größer ist als der mit der Referenzreihe, die sich auf die Tumorbereiche bezieht, als Nicht-Tumor, wenn es sich um einen der beiden anderen handelt;

420) in dem Fall, in dem keiner der Korrelationskoeffizienten größer als der erste vorgegebene Schwellenwert ist, oder in dem Fall, in dem die beiden niedrigeren Korrelationskoeffizienten nicht beide niedriger als der zweite vorgegebene Schwellenwert sind, wird die j-te Zone nicht klassifiziert und es werden zusätzliche Diagnosetests durchgeführt empfohlen.

**15.** Ultraschallvorrichtung (1) zur Durchführung einer diagnostischen Untersuchung der Brust zur Diagnose von Brustkrebs, nach Anspruch 10, **dadurch gekennzeichnet, dass** der genannte diagnostische Parameter eine Klassifizierung der j-ten Verdachtszone (Zj) als Tumor oder Nicht-Tumor ist, erhalten von:

500) Berechnung des Korrelationskoeffizienten des Durchschnittsspektrums in Bezug auf die j-te Zone mit

- das Referenztumorspektrum;
- das Referenzspektrum eines verdächtigen Bereichs, der sich später als Nicht-Tumor herausstellte;
- das in Nummer 360) berechnete Referenzspektrum für das äußere Gewebe der verdächtigen Bereiche;

510) für den Fall, dass einer der drei am Punkt 500 berechneten Koeffizienten größer als ein erster vorgegebener Schwellenwert ist und die anderen kleiner als ein zweiter vorgegebener Schwellenwert sind, Einstufung der j-ten Zone als Tumor, wenn der Korrelationskoeffizient größer als der mit dem Referenztumorspektrum ist, als Nicht-Tumor, wenn es sich um einen der beiden anderen handelt;

520) In dem Fall, in dem keiner der Korrelationskoeffizienten größer als der erste vorgegebene Schwellenwert ist, oder in dem Fall, in dem die beiden niedrigeren Korrelationskoeffizienten nicht beide niedriger als der zweite vorgegebene Schwellenwert sind, wird die j-te Zone nicht klassifiziert.

**16.** Ultraschallvorrichtung (1) zur Durchführung einer diagnostischen Untersuchung der Brust zur Diagnose von Brustkrebs, nach Anspruch 10, **dadurch gekennzeichnet, dass** als diagnostischer Parameter eine Klassifizierung des j-ten verdächtigen Bereichs als Tumor oder Nicht-Tumor gewonnen wird von:

600) Unterteilung der verfügbaren Referenzdatenreihen in eine Reihe von Trainingsdaten und eine Reihe von Validierungsdaten,

610) Trainieren eines neuronalen Klassifikationsnetzes unter Verwendung des Trainingssatzes, um einen Datensatz in Bezug auf einen verdächtigen Tumor oder Nicht-Tumorbereich zu klassifizieren,

620) Präsentation aller Daten bezüglich der j-ten Zone an das trainierte Netzwerk,

630) Klassifizierung der j-ten Zone als Tumor oder Nicht-Tumor je nach Netzwerkfluss.

**17.** Ultraschallvorrichtung (1) zur Durchführung einer diagnostischen Untersuchung der Brust zur Diagnose von Brustkrebs, nach Anspruch 15, **dadurch gekennzeichnet, dass** der diagnostische Parameter ein numerischer Wert der Wahrscheinlichkeit ist, dass die j-te verdächtige Zone (Zj) entweder vorhanden ein Tumor oder Nicht-Tumor ist, berechnet durch Umrechnung des Korrelationskoeffizienten des Durchschnittsspektrums in Bezug auf die j-te Zone mit dem Referenzspektrum einer Tumorzone, berechnet bei Punkt 500, in einen Prozentwert).

## Revendications

**1.** Appareil à ultrasons (1) pour réaliser un examen diagnostique du sein pour le diagnostic d'un cancer du sein comprenant deux sondes à ultrasons (10, 20),
**caractérisé en ce que**

chacune desdites sondes (10, 20) comprend un premier réseau (11, 21) de transducteurs piézoélectriques ou CMUT avec une première fréquence nominale (f1), et un deuxième réseau de transducteurs piézoélectriques ou CMUT (12, 22) avec une deuxième fréquence nominale (f2), lesdites sondes (10, 20) étant disposées à l'opposé l'une de l'autre, et configurées pour tourner le long d'une trajectoire circulaire ayant pour centre le milieu du segment les reliant et pour glisser l'une vers l'autre le long de ladite droite les reliant, de sorte qu'ils viennent en contact avec le sein de la patiente par des parties diamétralement opposées,

**en ce que** lesdits réseaux (11, 12, 21, 22) comprennent chacun une pluralité de transducteurs piézoélectriques ou CMUT, positionnés de manière à ce que les acquisitions soient réalisées sur un plan d'acquisition orthogonal au plan de ladite trajectoire circulaire,

et **en ce que** ledit appareil est configuré pour effectuer, en déplaçant lesdites sondes (10, 20) le long de ladite trajectoire circulaire, une pluralité de balayages par rapport à une pluralité de plans d'acquisition (P0, P1...) passant par ledit point milieu, orthogonaux à ladite trajectoire circulaire et tournés l'un par rapport à l'autre d'un angle ($\alpha$).

2. Appareil à ultrasons (1) pour réaliser un examen diagnostique du sein pour le diagnostic du cancer du sein, selon la revendication 1, **caractérisé en ce que** ledit dispositif comprend également un support circulaire (30),

et **en ce que** chaque sonde (10, 20) comprend également une tige de fixation (13, 23) associée audit support circulaire (30), de manière à ce que les deux sondes soient positionnées le long de la même direction, à l'opposé l'une de l'autre, avec les réseaux (11, 12, 21, 22) positionnés dans le même plan de l'axe de symétrie (a) dudit guide circulaire (30).

3. Appareil à ultrasons (1) pour réaliser un examen diagnostique du sein pour le diagnostic du cancer du sein, selon la revendication 1 ou 2, **caractérisé en ce que** la forme de la tête de chacune desdites sondes (10, 20) est concave, de sorte qu'une meilleure adhérence à la forme du sein est autorisée.

4. Appareil à ultrasons (1) pour réaliser un examen diagnostique du sein pour le diagnostic du cancer du sein, selon l'une quelconque des revendications précédentes, **caractérisé en ce que** ladite première fréquence nominale (f1) est choisie pour réaliser une imagerie à ultrasons en mode B démarrant à partir des signaux réfléchis par les tissus mammaires,

et ladite deuxième fréquence nominale (f2) est choisie pour effectuer des mesures d'atténuation acoustique, en travaillant en mode transmission avec le réseau de l'autre sonde ayant la même fréquence nominale.

5. Appareil à ultrasons (1) pour réaliser un examen diagnostique du sein pour le diagnostic du cancer du sein, selon l'une quelconque des revendications précédentes, **caractérisé en ce que** ladite première fréquence nominale (f1) est comprise entre 7 et 10 MHz et ladite deuxième fréquence nominale (f2) est comprise entre 1 et 3 MHz.

6. Appareil à ultrasons (1) pour réaliser un examen diagnostique du sein pour le diagnostic du cancer du sein, selon l'une quelconque des revendications précédentes, **caractérisé en ce que** chaque sonde comprend également des moyens de couplage acoustique (14, 15) avec la peau du sein, comprenant une membrane souple (14), remplie d'un gel (15), l'ensemble membrane (14) et gel (15) étant configuré pour être pressé lorsque la sonde est poussée vers le sein, s'adaptant ainsi à la forme du sein et garantissant ainsi un couplage mécanique pour la transmission des ultrasons.

7. Appareil à ultrasons (1) pour réaliser un examen diagnostique du sein pour le diagnostic de cancer du sein, selon l'une quelconque des revendications précédentes, comprenant en outre des moyens électroniques de calcul sur lesquels sont chargés des programmes informatiques configurés pour effectuer, pour chaque position d'acquisition, la procédure d'acquisition suivante :

100) acquisition d'une première image à ultrasons en mode B relative à la partie de tissus mammaires comprise entre ladite première sonde (10) et ledit axe de symétrie, au moyen dudit premier réseau (11) de ladite première sonde (10);

110) acquisition d'une deuxième image à ultrasons en mode B relative à la partie de tissus comprise entre ladite deuxième sonde (10) et ledit axe de symétrie, au moyen dudit premier réseau (21) de ladite deuxième sonde (20) ;

120) stockage des signaux ultrasonores réfléchis «bruts», tels que détectés par chaque sonde (10, 20) avant tout traitement ultérieur, notamment avant le traitement de création d'image en mode B correspondant;

130) émission d'une impulsion ultrasonore au moyen du premier transducteur piézoélectrique ou CMUT (121) dudit deuxième réseau (12) de ladite première sonde (10), et détection :

- du signal ultrasonore relatif transmis au moyen du premier transducteur piézoélectrique ou CMUT (221) dudit deuxième réseau (22) de ladite deuxième sonde (20) et

- du signal ultrasonore relatif réfléchi au moyen dudit premier transducteur piézoélectrique ou CMUT (121) dudit deuxième réseau (12) de ladite première sonde (10),

140) répétition de l'étape 130) pour chaque couple de transducteurs piézoélectriques ou CMUT desdits deuxièmes réseaux (12, 22) desdites première (10) et deuxième sondes.

8. Appareil à ultrasons (1) pour réaliser un examen diagnostique du sein pour le diagnostic du cancer du sein, selon la revendication 7, **caractérisé en ce qu'**il est configuré pour réaliser une pluralité d'acquisitions dans une série de plans d'acquisition (P0, P1), tournés l'un par rapport à l'autre selon un angle prédéfini ($\alpha$), de sorte qu'à chaque point de chaque plan d'acquisition soit associé:

- une valeur ($S_i$) relative à l'intensité du signal ultrasonore réfléchi,
- une valeur ($B_i$) de l'échelle de gris, associée à l'image à ultrasons en mode B, l'ensemble desdites valeurs ($Bi$) constituant ainsi un modèle à ultrasons 3D du volume mammaire, et **en ce que** pour chaque position d'acquisition sont mémorisés:
- des signaux ultrasonores bruts réfléchis en radiofréquence relatifs à l'ensemble des lignes de propagation du signal ultrasonore de chaque premier (11, 21) et deuxième réseau (12, 22),
- des signaux ultrasonores bruts transmis en radiofréquence, relatifs à toutes les lignes de propagation du signal ultrasonore desdits deuxièmes réseaux (12, 22).

9. Appareil à ultrasons (1) pour réaliser un examen diagnostique du sein pour le diagnostic du cancer du sein, selon la revendication 8, **caractérisé en ce que** lesdits programmes informatiques sont configurés pour réaliser un procédé de calcul d'un paramètre diagnostique représentatif de la présence ou non d'un cancer au sein comprenant les étapes suivantes:

200) segmentation dudit modèle à ultrasons 3D du volume mammaire pour individualiser la présence d'une ou plusieurs zones suspectes (Zj), **caractérisées par** une couleur plus claire (hyperéchogène) ou plus foncée (hypoéchogène) par rapport au tissu environnant, ou de toute façon **caractérisée par** toute autre inhomogénéité détectable au moyen d'une analyse en niveaux de gris,
201) calcul d'un paramètre diagnostique (Dj) relatif à chaque zone suspecte (Zj), indiquant la probabilité qu'une telle zone puisse être classée comme cancer du sein, ledit paramètre diagnostique (Dj) dépendant d'un ou plusieurs des facteurs suivants:

(i) des informations morphologiques dérivées de l'imagerie en mode B des points dudit modèle 3D à l'intérieur de la ième zone (Zj);
(ii) des informations relatives au coefficient d'atténuation acoustique moyen par rapport à au moins une partie de la ligne de propagation du signal ultrasonore contenu à l'intérieur de la jème zone (Zj);
(iii) des informations relatives au spectre de fréquence du signal associé à la jème zone (Zj).

10. Appareil à ultrasons (1) pour réaliser un examen diagnostique du sein pour le diagnostic du cancer du sein, selon la revendication 9, **caractérisé en ce que** ledit paramètre diagnostique est calculé au moyen du procédé selon les étapes suivantes de:

210) calcul d'une pluralité de caractéristiques morphologiques issues de l'imagerie à ultrasons de la jème zone (Zj), choisies dans la liste ci-dessous:

- volume de la zone;
- rapport surface/volume;
- dimension maximale;
- excentricité;
- rapport entre la valeur de gris moyenne de la jème zone (Zj) et la valeur de gris moyenne de la région environnante;

220) calcul du coefficient d'atténuation acoustique moyen (Aj) relatif à au moins une partie de la ligne de propagation du signal ultrasonore contenu à l'intérieur de la jème zone (Zj);
230) calcul du rapport entre ledit coefficient d'atténuation acoustique moyen et le coefficient d'atténuation acoustique moyen de la région extérieure à la jème zone (Ats);
235) calcul de la vitesse de propagation acoustique du signal ultrasonore relative à au moins une ligne de propagation du signal ultrasonore traversant la jème zone (Zj) et à au moins une ligne de propagation du signal ultrasonore ne traversant aucune zone suspecte;

260) calcul d'au moins un spectre de fréquence du signal ultrasonore brut en radiofréquence émis par l'un desdits premiers réseaux (11, 21) et réfléchi par une partie de tissu correspondant à un segment d'une ligne de propagation du signal ultrasonore contenu à l'intérieur de la jème zone;

270) calcul d'au moins un paramètre quantitatif extrait par le spectre du point 260), choisi dans la liste comprenant les paramètres suivants:

- valeur moyenne du spectre;
- zone sous-tendue par le spectre dans un intervalle de fréquence déterminé et/ou un intervalle d'intensité déterminé;
- largeur du spectre dans un intervalle de fréquence prédéterminé;
- valeur de fréquence correspondant à la valeur maximale dudit spectre;
- pente d'une ligne interpolant une pluralité de points dudit spectre dans un intervalle de fréquence prédéterminé;
- coefficients d'un polynôme interpolant les points dudit spectre dans un intervalle de fréquence contenant le maximum dudit spectre;

280) calcul d'un paramètre de diagnostic fonction de la comparaison d'au moins un des paramètres calculés aux étapes 210 à 270 avec des paramètres respectifs obtenus au moyen d'examens à ultrasons effectués au moyen de l'appareil selon l'une quelconque des revendications précédentes et référencé à:

- zones suspectes pour lesquelles la présence de la pathologie a été ultérieurement confirmée par un examen histologique;
- zones suspectes pour lesquelles la présence d'un cancer du sein a été ultérieurement exclue au moyen d'une biopsie ou d'une autre technique fiable équivalente;
- portions de tissus non intéressées par les zones suspectes.

**11.** Appareil à ultrasons (1) pour effectuer un examen diagnostique du sein pour le diagnostic du cancer du sein, selon la revendication 10, **caractérisé en ce qu'**au point 260) un spectre de fréquences associé à la jème zone (Zj) est calculé comme moyenne d'une pluralité de spectres par rapport aux segments de propagation respectifs du signal contenu à l'intérieur de ladite zone suspecte (Zj), et **en ce que** pour le calcul du spectre moyen, la procédure de sélection des spectres suivante est effectuée, à utiliser pour le calcul du spectre moyen par rapport à la jème zone:

- calcul du coefficient de corrélation du spectre moyen avec l'ensemble des spectres utilisés pour son calcul;
- exclusion des spectres pour lesquels un tel coefficient de corrélation est inférieur à un seuil prédéterminé;
- calcul d'un nouveau spectre moyen par rapport à la jème zone;
- répétition de la procédure de calcul de corrélation et d'exclusion des spectres, jusqu'à ce que tous les spectres restants présentent un coefficient de corrélation avec le spectre moyen supérieur ou égal audit seuil prédéterminé.

**12.** Appareil à ultrasons (1) pour réaliser un examen diagnostique du sein pour le diagnostic du cancer du sein, selon la revendication 11, **caractérisé en ce que** ladite comparaison du point 280) s'effectue vers le bas de la procédure de définition suivante d'ensembles de valeurs de référence tumorales, non tumorales et saines, détecté lors d'examens à ultrasons effectués au moyen de l'appareil selon l'une quelconque des revendications précédentes chez des patients pour lesquels la présence de la pathologie tumorale a été ultérieurement confirmée ou exclue au moyen d'un examen histologique:

300) définition, pour chaque paramètre calculé aux points 210 à 250, d'une valeur tumorale moyenne (VMt), et d'un intervalle de confiance relatif à 75% et 95%, calculé à partir de la répartition statistique des valeurs de chaque paramètre pour tous les zones suspectes dont la nature tumoral a été confirmée;

310) définition, pour chaque paramètre calculé aux points 210 à 250, d'une valeur moyenne non tumorale (VMnt), et d'un intervalle de confiance relatif à 75% et 95%, calculé à partir de la répartition statistique des valeurs de chaque paramètre pour toutes les zones suspectes dont la nature tumorale (Znt) a été exclue;

320) définition, pour chaque paramètre calculé aux points 210 à 250, d'une valeur moyenne saine (VMs), et d'un intervalle de confiance relatif à 75% et 95%, calculé à partir de la répartition statistique des valeurs de chaque paramètre pour tous les portions de tissus appartenant audit ensemble de portions de tissus sains non intéressées par les zones suspectes visibles aux ultrasons (Tnz);

340) sélection d'une pluralité de segments de propagation du signal ultrasonore:

- contenu à l'intérieur des zones tumorales;
- contenu à l'intérieur de zones suspectes, qui par la suite n'a pas donné lieu à une tumeur;
- contenu dans des tissus situés en dehors des zones suspectes;

350) calcul de la transformée en fréquence du signal ultrasonore brut en radiofréquence associé à chacun desdits segments de propagation du signal ultrasonore;

360) calcul:

- du spectre tumoral de référence obtenu comme spectre moyen relatif à l'ensemble des signaux ultrasonores relatifs aux zones tumorales;
- du spectre de référence non tumoral obtenu comme spectre moyen relatif à l'ensemble des signaux ultrasonores relatifs aux zones suspectes, résultant ultérieurement non tumorales;
- du spectre de référence relatif aux tissus externes des zones suspectes obtenu comme spectre moyen relatif à l'ensemble des signaux ultrasonores relatifs aux tissus externes des zones suspectes.

**13.** Appareil à ultrasons (1) pour réaliser un examen diagnostique du sein pour le diagnostic d'un cancer du sein, selon la revendication 10, **caractérisé en ce que** ladite procédure de définition d'ensembles de valeurs tumorales de référence comprend l'étape de:

330) sélection des paramètres pour lesquels la valeur tumorale moyenne (VMt) est en dehors à la fois de l'intervalle de confiance à 75 % de la valeur moyenne relative saine (VMs), et de l'intervalle de confiance à 75 % de la valeur moyenne relative non tumorale (VMnt).

**14.** Appareil à ultrasons (1) pour réaliser un examen diagnostique du sein pour le diagnostic du cancer du sein, selon la revendication 10, **caractérisé en ce que** ledit paramètre diagnostique est une classification de ladite jème zone suspecte (Zj) comme «tumorale» ou «non tumorale», obtenu par:

400) calcul du coefficient de corrélation de l'ensemble des paramètres relatifs à la jème zone

- avec la série de référence relative aux zones tumorales,
- avec la série de référence relative aux zones suspectes, avec un résultat non tumoral dans les analyses ultérieures;
- avec la série de référence relative aux tissus n'appartenant pas aux zones suspectes,

410) dans le cas où l'un des trois coefficients calculés au point 400) est supérieur à un premier seuil prédéterminé et les autres sont inférieurs à un deuxième seuil prédéterminé, classement de la jème zone comme tumorale si le coefficient de corrélation est supérieur à celui avec la série de référence relative aux zones tumorales, comme non tumorales s'il s'agit de l'une des deux autres;

420) dans le cas où aucun des coefficients de corrélation n'est supérieur audit premier seuil prédéterminé, ou dans le cas où les deux coefficients de corrélation inférieurs ne sont pas tous deux inférieurs audit deuxième seuil prédéterminé, la jème zone n'est pas classée et des examens diagnostiques supplémentaires sont recommandés.

**15.** Appareil à ultrasons (1) pour réaliser un examen diagnostique du sein pour le diagnostic du cancer du sein, selon la revendication 10, **caractérisé en ce que** ledit paramètre diagnostique est une classification de la jème zone suspecte (Zj) comme tumorale ou non tumorale, obtenue par:

500) calcul du coefficient de corrélation du spectre moyen par rapport à la jème zone avec

- le spectre tumoral de référence;
- le spectre de référence d'une zone suspecte, ultérieurement révélée non tumorale;
- le spectre de référence relatif aux tissus externes des zones suspectes calculé au point 360);

510) dans le cas où l'un des trois coefficients calculés au point 500 est supérieur à un premier seuil prédéterminé et les autres sont inférieurs à un deuxième seuil prédéterminé, classement de la jème zone comme tumorale si le coefficient de corrélation est supérieur à celui avec le spectre tumoral de référence, comme non tumoral s'il s'agit de l'un des deux autres;

520) dans le cas où aucun des coefficients de corrélation n'est supérieur audit premier seuil prédéterminé, ou dans le cas où les deux coefficients de corrélation inférieurs ne sont pas tous deux inférieurs audit deuxième seuil prédéterminé, la jème zone n'est pas classée.

**16.** Appareil à ultrasons (1) pour réaliser un examen diagnostique du sein pour le diagnostic du cancer du sein, selon la revendication 10, **caractérisé en ce que** ledit paramètre diagnostique est une classification de la jème zone suspecte comme tumorale ou non tumorale, obtenue par:

600) subdivision des séries de données de référence disponibles en une série de données d'apprentissage et une série de données de validation,
610) apprentissage d'un réseau neuronal de classification, en utilisant ledit ensemble d'apprentissage, pour classer un ensemble de données par rapport à une zone suspecte tumorale ou non tumorale,
620) présentation de l'ensemble des données relatives à la jème zone au réseau entraîné,
630) classification de la jème zone comme tumorale ou non tumorale en fonction du débit du réseau.

**17.** Appareil à ultrasons (1) pour réaliser un examen diagnostique du sein pour le diagnostic du cancer du sein, selon la revendication 15, **caractérisé en ce que** ledit paramètre diagnostique est une valeur numérique de la probabilité que ladite jème zone suspecte (Zj) soit tumorale ou non tumorale, calculé en convertissant en valeur en pourcentage ledit coefficient de corrélation du spectre moyen relatif à la jème zone avec le spectre de référence d'une zone tumorale calculé au point 500).

Fig. 1

Fig, 2

Fig, 3

Fig. 4

Fig. 5

Fig. 6

Fig. 7

200 — SEGMENTATION

SPOTS EXTRACTION

M1   M2   M3   ......   Mj

210-250 — PARAMETERS CALCULATION

MORPHOLOGICAL PARAMETERS

ATTENUATION PARAMETERS

BUB

IRC

260 — AVERAGE SPECTRUM CALCULATION

AVERAGE SPECTRUM

**Fig. 8**

Fig. 9

Fig. 10

51

50

1

Fig. 11

Fig. 12

**REFERENCES CITED IN THE DESCRIPTION**

**Patent documents cited in the description**

- CN 110680380
- WO 02089672 A
- EP 2868279 A
- US 20130041261 A
- US 2012029358 A
- US 2004064046 A
- CN 111213065
- US 2013041260 A
- US 4222274 A
- US 10285667 B